# EUROPEAN PATENT SPECIFICATION

(11) **EP 2 653 101 B1**
(45) Date of publication and mention of the grant of the patent: **25.11.2015**
(21) Application number: 11848988.9
(22) Date of filing: 20.09.2011
(51) Int. Cl.: A61B 5/151

(54) **LANCET DEVICE**
LANZETTENVORRICHTUNG
DISPOSITIF À LANCETTE

(30) Priority: 15.12.2010 KR 20100128560
(43) Date of publication of application: 23.10.2013
(73) Proprietor: i-Sens, Inc., Nowon-gu, Seoul 139-050 (KR)
(72) Inventor: CHA, Geun Sig, Seoul 120-100 (KR); NAM, Hakhyun, Seoul 142-063 (KR); CHA, Eun-Jong, Cheongju-si Chungcheongbuk-do 361-753 (KR); KIM, Kyung-Ah, Cheongju-si Chungcheongbuk-do 361-765 (KR)
(74) Representative: Modiano, Micaela Nadia
(86) International application number: PCT/KR2011/006936
(87) International publication number: WO 2012/081809

(56) References cited:
- EP-A2- 1 090 584
- EP-A2- 1 779 780
- WO-A1-2009/022136
- WO-A2-2005/023088
- DE-A1-102004 025 651
- JP-A- 8 276 013
- JP-A- 2007 503 913
- KR-A- 20050 032 194
- KR-A- 20080 104 305
- US-A1- 2004 162 573

## Description

### Field of the Invention

The present invention relates to a lancet device. More particularly, the present invention relates to a lancet device that can solve the problem of falling or losing a cover when mounting or separating a lancet and reduce the pain that accompanies blood-taking.

### Description of the Related Art

In general, chronic diabetics have to measure the blood glucose level by performing a blood sugar test by themselves everyday at home and to perform disease control in order to keep a predetermined blood glucose level.

They have to collect blood for the blood glucose test, and this case, generally, they stick a disposable lancet into the skin of a portion, usually a finger, of their bodies, take and put a small amount of capillary blood onto a strip, and then measure the blood glucose level using a blood glucose meter with the strip mounted.

A lancing device is generally used as the device for taking blood.

The lancing device is composed of a lancet holder mounted with a disposable lancet, a cover that covers a lancet and has a hole through which only the tip of a needle protrudes to penetrate a skin, and a spring and a releasing member that provide a penetration force. The disposable lancet has a needle at one end of a lancet body and a protection cap is combined with the needle.

According to the lancing devices having this configuration in the related art, a user removes the cover from a lancing device, mounts a disposable lancet onto the lancet holder, attaches the cover with the spring compressed, brings the lancet in close contact with a portion with many capillaries such as fingers, and then releases the disposable lancet by pulling a releasing switch, such that the lancet penetrates the skin.

However, it is troublesome to use the lancing devices, because it is required to separate a cover from the lancing devices and place it at a side or hold it for a moment, when mounting or separating a lancet, and the cover is small, such that the cover is likely to be lost when a user falls it by mistake.

Further, since the needle penetrates a skin, a pain accompanies, but it is taken as a matter of course to be accepted in order to take blood.

Examples of lancet devices are known from the following documents.

Document EP 1 779 780 discloses a connector engaged with a lancing apparatus and an integrated medical device. The integrated medical device includes a test strip and a tissue penetration member with lancet. A firing mechanism is configured for producing a firing force in a first direction. A lancing mechanism is configured for delivering a lancing force in a second direction with the second direction being toward a target site and in opposition to the first direction. A linkage arm is pivotably attached to an inner housing and has first and second ends engaged to the firing and lancing mechanisms, respectively. During use, pivoting of the linkage arm converts the firing force in the first direction into the lancing force in the opposing second direction.

Document US 6 409 740 discloses a blood lancet device for withdrawing blood from a body part for diagnostic purposes. The device is provided with a lancet holder and a tip that protrudes out of an outlet opening of a housing. A lancet drive has a drive rotor comprising a pressure surface having a tensioning section preceding a vertex against the sense of rotation, and having an increasing center distance against the sense of rotation. The pressure element moves along the tensioning section during tensioning of the lancet drive.

Document WO 2009/022136 discloses a lancing device including a main barrel portion to the front end of which is hinged a forward portion for movement between the open position and a closed position. The main barrel portion contains a drive mechanism, cocking slider and trigger, whilst the forward portion contains a lancet holder into which a lancet may be loaded front end first. Also disclosed is a lancing device having a main housing having a forward nose portion to the rear of which is a flip up lancet hatch into which a lancet may be loaded front end first.

### Disclosure

### Technical Problem

The present invention has been made in an effort to provide a lancet device having advantages of being able to solve the problem of falling or losing a cover when mounting or separating a lancet and reduce the pain that accompanies blood-taking.

### Technical Solution

An exemplary embodiment of the present invention provides a lancet device, which includes: a lancet holder having a lancet seat to mount a lancet at one end; an operating mechanism disposed at the other end of the lancet holder and loading and releasing the lancet holder along a predetermined path;
and a housing receiving the operating mechanism that has one end coupled to the lancet holder, and further comprises a cover that covers the housing so that the lancet holder is received in the housing, is at least partially connected with the housing in order not to separate from the housing in use, the cover having protrusions on the front part and a through-hole at the center through which a lancet needle of the lancet instantaneously protrudes and a coupling portion that hinges the cover to one end of the housing, the protrusions being radially formed at regular angles, alternately arranged on a plurality of coaxial circles, having a triangular shape, and being alternately arranged in two lines.

According to the present invention described above, the following effects are achieved.

First, it is possible to remove the problem in that a user has to separate the cover from the lancet device and then places it or hold it in a hand or the problem of losing the cover while mounting and separating the lancet.

Second, a user can sensuously feel the position to take blood from, by means of the protrusions, and easily takes blood because the blood vessels around the portion to take blood from are pressed and blood is collected, and the pain reduces when the lancet is released.

Third, since the protrusions are alternately arranged along a plurality of coaxial circles and the contact area is wide, it is possible to stimulate many nerves around the portion to take blood from at a time, such that it is possible to press more blood vessels around the portion and is easy to take more blood.

### Description of Drawings

FIG. 1 is a perspective view of a lancet device according to an exemplary embodiment of the present invention.
FIG. 2 is an exploded perspective view of the lancet device according to an exemplary embodiment of the present invention.
FIG. 3 is a cross-sectional view of the assembly of the parts shown in FIG. 1.
FIG. 4 is a partial enlarged view of FIG. 1.
FIG. 5 is a status view for illustrating how to use a cover of the lancet device according to an exemplary embodiment of the present invention.

### Best Mode

Hereinafter, exemplary embodiments of the present invention will be described in detail with reference to the accompanying drawings. First, when giving the components in the drawings reference numerals, it should be noted that the same reference numerals are given to reference numerals as same as possible even if they are shown in different drawings. Further, the in description of the present invention, the detailed description of related well-known configurations and functions is not provided, when it is determined as making the scope of the present invention unclear.

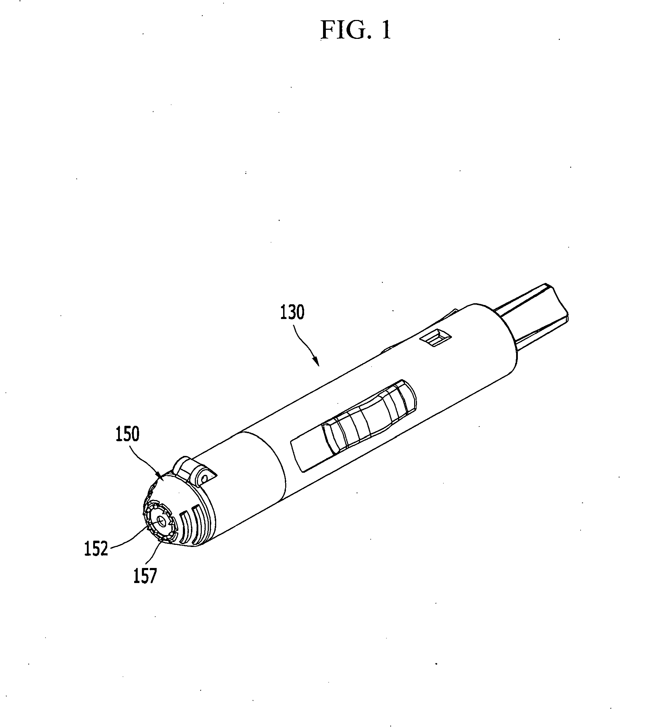

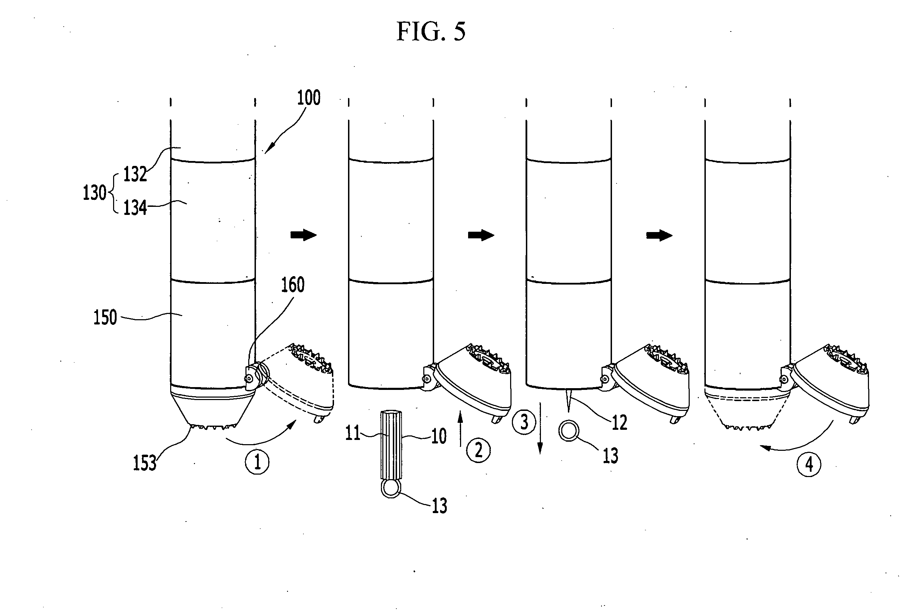

Referring to FIGS. 1 and 5, a lancet device 100 according to an exemplary embodiment of the present invention includes a lancet holder 110, an operating mechanism 120, a housing 130, an operation switch 170, a cover 150, a coupling portion 160, and a depth adjusting member 140.

The lancet holder 110 has a lancet seat 111 to mount a lancet 10 at one end and is disposed at a side in the housing 130.

The lancet 10, which is disposable, has a lancet needle 12 at one end of a lancet body 11 with a predetermined length and the lancet needle 12 is combined with a protection cap 13 (see FIG. 5).

The protection cap 13 is coupled to one end of the lancet needle 12 by insert injection, and according to this configuration, the protection cap 13 can be separated from the lancet needle 12 even by a small force.

The operating mechanism 120 is disposed at the other end of the lancet holder 110 and has a structure for loading and releasing the lancet holder 110 along a predetermined path, and for this operation, it includes a plurality of springs 121, 122, and 123, as shown in FIG. 1.

The housing 130, a cylinder sized to be portable by a user, receives the operating mechanism 120, with the lancet holder 110 coupled to one end, and the operation switch 170 operating with the operating mechanism 120 is disposed at a predetermined position.

The operation switch 170 is connected to the operating mechanism 120 and operates the operating mechanism 120 by being pressed.

When a user presses the operation switch 170, the springs 121, 122, and 123 are instantaneously compressed, and extend and hit a movable shaft 125 under which a shock transmission rod 124 is positioned, the movable shaft 125 hits the lancet holder 110 holding the lancet 10, and the lancet 10 protrudes outward.

The cover 150 covers an end of the housing 130, has a through-hole 152 at the center through which the lancet needle 12 of the lancet 10 instantaneously protrudes, and has a simple opening/closing structure such as a hinged door, different from the way of mounting a lancet after separating a cover in the existing products.

The coupling portion 160 hinges one end of the housing 130 with the cover 150, such that the cover 150 pivots to open/close at 180 degrees, as shown in FIG. 5.

According to this structure, it is possible to remove the problem in that a user has to separate the cover 150 from the lancet device 100 and then places it or hold it in a hand or the problem of losing the cover 150 while mounting and separating the lancet 10.

To this end, the cover has a front part 151 and a locking protrusion 155.

The through-hole 152 is formed at the center of the front part 151 so that the lancet needle 12 can instantaneously protrude out of the cover 150 and take blood by means of the operating mechanism 120.

The locking protrusion 155 is formed at the other end of the front part 151 in the shape corresponding to a fitting groove 131 formed at one end of the housing 130.

Therefore, it is possible to keep the cover 150 closed by fitting the locking protrusion 155 into the fitting groove 131 without adding a complicated configuration.

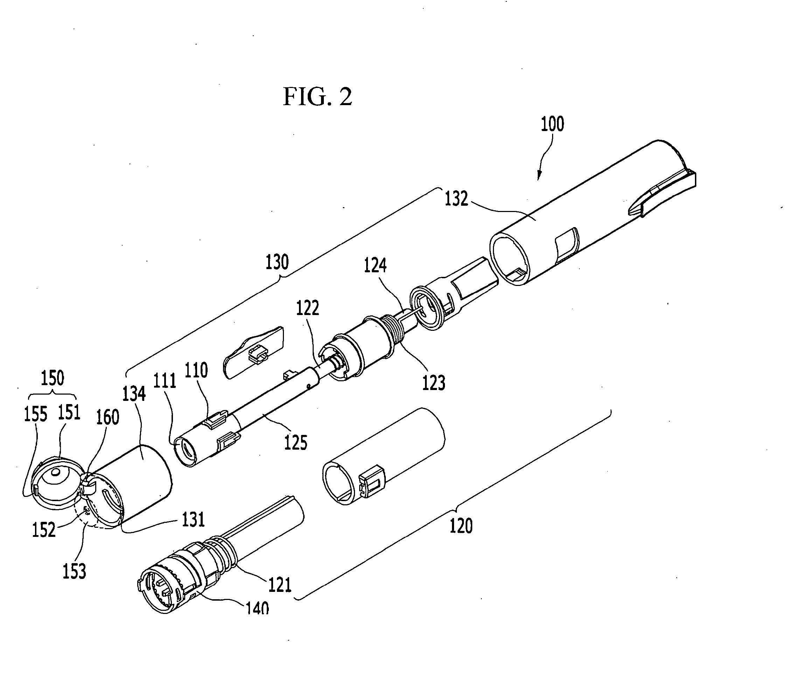

Further, as shown in FIGS. 1 and 2, a plurality of protrusions 153 is radially formed at regular angles in the shape of a gear on the front part 151 of the cover 150.

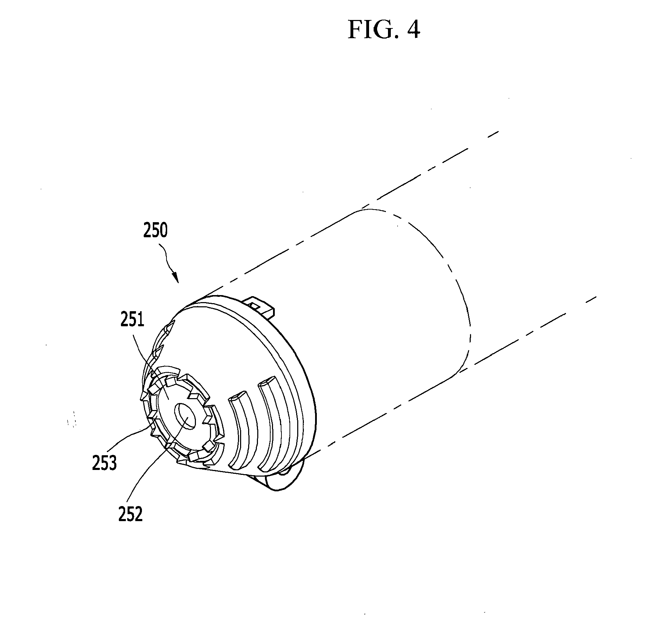

In particular, as shown in FIG. 4, it is preferable that the protrusions 153 have a sharp triangular shape and are arranged along a plurality of coaxial circles formed along the edge of the through-hole 152 of the front part 151 of the cover 150, that is, alternately in two lines to increase the area that comes in contact with a skin.

The protrusions 153 press the skin and stimulate the nerves of the portion to take blood from, before the lancet needle 12 penetrates the skin in taking blood.

Therefore, a user can sensuously feel the position to take blood from, and easily takes blood because the blood vessels around the portion to take blood from are pressed and blood is collected, and the pain reduces when the lancet 10 is released.

The depth adjusting member 140 adjusts the penetration depth under the skin of the lancet needle 12.

To this end, as shown in FIG. 1, the housing 130 includes a first housing part 132 receiving the operating mechanism 120 and a second housing part 134 rotating about the axis of the first housing part 132 and combined with the depth adjusting member 140 therein.

The second housing part 132 is combined with the cover 150 and the depth adjusting member 140 and the distance between the through-hole 152 and the lancet needle 12 is adjusted by rotating the cover 150 or the second housing part 132.

How to use the cover 150 of the lancet device 100 of the present invention is described hereafter with reference to FIG. 5.

First, a user opens the cover 150 from the lancet device 100 by holding and turning the cover 150 by 180 degrees to the coupling portion 160 (①) and then mounts the lancet 10 onto the lancet seat 111 (see FIG. 2) of the lancet holder 110 (②),

Then, the user separates the protection cap 13 from the lancet 10 to expose the lancet needle 12 to the outside (③), and then mounts the cover 150 to one end of the housing 130 by turning the cover 150 by 180 degrees to the other side of the coupling portion 160 (④).

The user brings the lancet device 100 ready for taking blood in contact with a portion to take blood from, such as a finger, in which the protrusions 153 press the area around the portion, such that nerves around are stimulated.

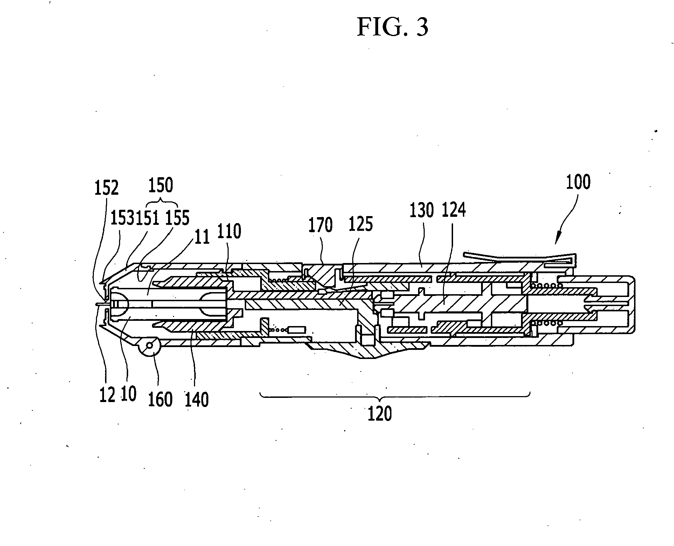

Finally, when the user operates the operating mechanism 120 by pressing the operation switch 170 (see FIG. 3), the lancet 10 is released and the lancet needle 12 penetrates the skin, thereby taking blood.

The process of opening the cover 150 to remove the lancet 10 from the lancet device 100 after taking blood is also performed in the same way.

The above description is an example of an exemplary embodiment of the present invention and may be changed and modified in various ways by those skilled in the art without departing from the scope of the present invention. Therefore, the exemplary embodiments described herein are for explaining the present invention and the scope of the present invention is not limited to the exemplary embodiments. The protective range of the present invention should be construed by claims and the equivalents should be construed as being included in the scope of the present invention.

While this invention has been described in connection with what is presently considered to be practical exemplary embodiments, it is to be understood that the invention is not limited to the disclosed embodiments, but, on the contrary, is intended to cover various modifications and equivalent arrangements included within the scope of the appended claims.

## Claims

1. A lancet device (100) that includes:
a lancet holder (110) having a lancet seat (111) to mount a lancet (10) at one end;
an operating mechanism (120) disposed at the other end of the lancet holder (110) and loading and releasing the lancet holder (110) along a predetermined path; and
a housing (130) receiving the operating mechanism (120) that has one end coupled to the lancet holder (110),
the device comprising a cover (150) that covers the housing (130) so that the lancet holder (110) is received in the housing (130), is at least partially connected with the housing (130) in order not to separate from the housing (130) in use, **characterized in that** the cover (150) has protrusions (153) on the front part (151) and a through-hole (152) at the center through which a lancet needle (12) of the lancet (10) instantaneously protrudes, and a coupling portion (160) that hinges the cover (150) to one end of the housing (130),
and wherein the protrusions (153) are radially formed at regular angles, are alternately arranged on a plurality of coaxial circles, have a triangular shape, and are alternately arranged in two lines.

2. The device of claim 1, wherein the cover (150) has a locking protrusion (155) that is formed at the other end of the front part (151) and fitted in a fitting groove (131) formed at one end of the housing (130).

## Patentansprüche

1. Eine Lanzettenvorrichtung (100), die Folgendes einschließt:
einen Lanzettenhalter (110) mit einem Lanzettensitz (111) zur Montage einer Lanzette (10) an einem Ende,
einen Bedienmechanismus (120), angebracht am anderen Ende des Lanzettenhalters (110), der den Lanzettenhalter (110) entlang einem vordefinierten Pfad lädt und löst, und
ein Gehäuse (130), das den Bedienmechanismus (120) aufnimmt und, wobei ein Ende mit dem Lanzettenhalter (110) gekoppelt ist,
wobei die Vorrichtung eine Abdeckung (150) umfasst, die das Gehäuse (130) so abdeckt, dass der Lanzettenhalter (110) im Gehäuse (130) untergebracht ist, zumindest teilweise mit dem Gehäuse (130) verbunden ist, um sich im Gebrauch nicht vom Gehäuse (130) zu lösen, **dadurch gekennzeichnet, dass** die Abdeckung (150) Vorsprünge (153) am vorderen Teil (151) und eine Durchgangsbohrung (152) in der Mitte hat, durch welche eine Lanzettennadel (12) der Lanzette (10) instantan vorsteht, und einen Kopplungsabschnitt (160), der die Abdeckung (150) gelenkig mit einem Ende des Gehäuses (130) verbindet,
und wobei die Vorsprünge (153) radial in gleichmäßigen Winkeln geformt sind, abwechselnd auf einer Vielzahl koaxialer Kreise angeordnet sind, eine dreieckige Form haben und die abwechselnd in zwei Reihen angeordnet sind.

2. Die Vorrichtung gemäß Anspruch 1, wobei die Abdeckung (150) einen Blockiervorsprung (155) hat, der am anderen Ende des vorderen Teils (151) geformt ist und in eine Passnut (131) eingepasst ist, die an einem Ende des Gehäuses (130) geformt ist.

## Revendications

1. Dispositif à lancette (100) qui comprend:
un support de lancette (110) comportant un siège de lancette (111) pour monter une lancette (10) à une extrémité;
un mécanisme d'actionnement (120) disposé à l'autre extrémité du support de lancette (110) et qui charge et libère le support de lancette (110) le long d'un trajet prédéterminé; et
un boîtier (130) recevant le mécanisme d'actionnement (120) qui présente une extrémité couplée au support de lancette (110),
le dispositif comprenant un couvercle (150) qui couvre le boîtier (130) de sorte que le support de lancette (110) est reçu dans le boîtier (130), est au moins partiellement relié avec le boîtier (130) afin de ne pas se séparer du boîtier (130) en cours d'utilisation, **caractérisé en ce que** le couvercle (150) présente des saillies (153) sur la partie avant (151) et un trou traversant (152) au centre à travers lequel une aiguille de lancette (12) de la lancette (10) fait saillie instantanément, et une partie d'accouplement (160) qui articule le couvercle (150) à une extrémité du boîtier (130),
et dans lequel les saillies (153) sont formées radialement à des angles réguliers, sont agencées de manière alternée sur une pluralité de cercles coaxiaux, ont une forme triangulaire, et sont agencées de manière alternée sur deux lignes.

2. Dispositif selon la revendication 1, dans lequel le couvercle (150) présente une saillie de verrouillage (155) qui est formée à l'autre extrémité de la partie avant (151) et montée dans une rainure de montage (131) formée à une extrémité du boîtier (130).
